# EUROPEAN PATENT APPLICATION

(11) **EP 4 193 847 A1**
(43) Date of publication of application: **14.06.2023**
(21) Application number: 22210450.7
(22) Date of filing: 30.11.2022
(51) Int. Cl.: A23L 33/00, A23L 33/125, A23L 33/175, A23L 33/19, A23C 9/20

(54) **NUTRIENT COMPOSITION, FOOD INCLUDING THE SAME AND USE OF THE NUTRIENT COMPOSITION**

(30) Priority: 13.12.2021 CN 202111568623
(71) Applicant: Heilongjiang Feihe Dairy Co., Ltd., Qiqihar City, Heilongjiang 1648000 (CN)
(72) Inventor: XIE, Qinggang, QIQIHAR CITY, 164800 (CN); CUI, Dongying, QIQIHAR CITY, 164800 (CN); JIANG, Shilong, QIQIHAR CITY, 164800 (CN); LIANG, Aimei, QIQIHAR CITY, 164800 (CN); ZHANG, Yongjiu, QIQIHAR CITY, 164800 (CN); CAI, Fangliang, QIQIHAR CITY, 164800 (CN); LENG, Youbin, QIQIHAR CITY, 164800 (CN); LU, Siyu, QIQIHAR CITY, 164800 (CN)
(74) Representative: Hirsch & Associés

(57) **Abstract**

The invention relates to a nutrient composition, a food including the same and use of the nutrient composition. The nutrition composition includes: human milk oligosaccharide, preferably neutral fucosylated human milk oligosaccharide, which is preferably one or more selected from the group consisting of : 2'-fucosyl lactose, 3'-fucosyl lactose, lacto-N-fucopentaose I, lacto-N-difucohexaose I , lacto-N-difucohexaose II, preferably 2'-fucosyl lactose (2'-FL); milk fat globule membrane; and choline and/or an edible choline derivative. The nutritional composition can improve the brain development and intelligence of babies, infants and young children, especially promote neurodevelopment such as neuron maturation, synaptogenesis and myelination.

## Description

### Field of the Invention

The present invention generally relates to the field of food. Specifically, the present invention relates to a nutrient composition for improving the brain development and intelligence of infants and young children, especially promoting neurodevelopment such as neuron maturation, synaptogenesis and myelination etc., a food containing the nutrient composition, and use of the nutrient composition. More specifically, the present invention relates to a nutrient composition comprising human milk oligosaccharide (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), for example 2'-fucosyl lactose (2'-FL)), milk fat globule membrane, and choline and/or edible choline derivative; food comprising the nutrient composition, and the use of said nutrient composition for non-curing purposes (i.e., non-medical purpose) of improving the brain development and intelligence of infants and young children, especially promoting the neurodevelopment of infants and young children.

### Background of the Invention

Oligodendrocyte precursor cells (OPCs) were first discovered by Raff, and Miller et al. in 1993, and have been intensively studied. Central nervous systems, both under development and mature, contain oligodendroglia precursor cells. Myelin formed cells in the central nervous system originates from oligodendroglia precursor cells. The types of mylin protein expressed by oligodendrocytes, such as myelin-associated glycopeptides (MAGs) and myelin binding proteins (MBPs), correlate to their phase of maturation. Myelinating OLs express MAG, with the expression of MAG gradually increasing during the maturation of OLs. MAG is a sialic acid binding immunoglobulin-like lectin. Although MAG represents only a small proportion of the total protein content of myelin sheaths, it is expressed mainly in the periaxonal regions of the myelin sheaths. It seem to play an important role in oligodendrocyte- axon interaction and mediates bidirectional signalling between axons and OLs, in order to support the formation of the myelin sheaths. MBP is strongly expressed in mature myelinating OLs, and is one of the major components of myelin sheaths. MBP seems to have an active role in the formation and compaction of myelin sheaths. Indeed, MBP is polymerizing and forming a cohesive mesh-like protein network, which is essential for saltatory current.

Brain development is influenced by genetic and environmental factors. Amongst the latter, maternal and early-life nutrition play a key role in the process of neurodevelopment such as neuron maturation, synaptogenesis and myelination. Myelination is the process by which the oligodendrocyte (OL) in the central nervous system (CNS) form a myelin sheath around axons, which is critical for proper brain connectivity. In humans, myelination starts at mid-gestation, peaks during the first years of life. Environmental factors might influence myelination during human brain development. Specifically, different nutients exhibit different influence on myelination, suggesting that early-life nutrition might be of importance for the regulation of myelination.

During development of central nervous system, oligodendrocyte precursor cells (OPCs) migrate from within the cortex, and will generate the adult population of oligodendrocytes (OLs). After mitosis, OPCs differentiate into myelinating OLs. These OLs extend numberous processes to establish contacts with axons from different neurons to initiate myelination, which process enhances neuronal connectivity and supports the maturation of emerging cognitive function.

In the central nervous system, each step during myelination, including proliferation of OPCs, differentiation and maturation of OPCs into myelinating OLs, as well as myelin sheath formation is highly regulated by extrinsic and intrinsic factors. Particularly, various nutrients show different influences on myelination, suggesting that early-life nutrition might be of importance for the regulation of myelination. Therefore, identification of myelination-supporting early-life nutrition factors is of critical importance for optimal brain and cognition development.

There is a need for providing a composition which enables improving the brain development and intelligence of infants and young children, especially promoting neurodevelopment such as neuron maturation, synaptogenesis and myelination.

### Summary of the Invention

One object of the present incention is to provide a composition which enables improving the brain development and intelligence of infants and young children, especially promoting neurodevelopment such as neuron maturation, synaptogenesis and myelination.

Another object of the present invention is to provide a food containing the nutrient composition.

Another object of the present invention is to provide a use of said nutrient composition or food for non-curing purposes (i.e., non-medical purpose) of improving the brain development and intelligence of infants and young children, especially promoting neurodevelopment such as neuron maturation, synaptogenesis and myelination, in particular promoting the oligodendrocyte precursor cell proliferation, maturation, and differentiation into oligodendrocytes and/or myelination of oligodendrocytes.

The inventors have conducted studies and discovered that neurodevelopment such as neuron maturation, synaptogenesis and myelination can be significantly promoted and brain development and intelligence can be improved, by the use of human milk oligosaccharide (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), for example 2'-fucosyl lactose (2'-FL)), milk fat globule membrane, and choline and/or edible choline derivative in combination. Particularly, the inventors have discovered that human milk oligosaccharide (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), for example 2'-fucosyl lactose (2'-FL)), milk fat globule membrane, and choline and/or edible choline derivative can synergistically promote neurodevelopment such as neuron maturation, synaptogenesis and myelination, in particular can synergistically promote OPC proliferation, maturation and differentiation into mature OL, and/or OL myelination characteristics, and synergistically promote brain development of organics and improvement in memorization.

Particularly, the present invention is realized by:
1. A nutrient composition comprising:
   - human milk oligosaccharide (HMO), preferably neurtral fucosylated human milk oligosaccharide (HMO), preferably one or more selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), preferably 2'-fucosyl lactose;
   - milk fat globule membrane; and
   - choline and/or edible choline derivative, preferably the edible choline derivative being selected from the group consisting of choline chloride and/or choline bitartrate.
2. The nutrient composition of item 1, consisting of the human milk oligosaccharide; the milk fat globule membrane; and the choline and/or edible choline derivative.
3. The nutrient composition of either of items 1 and 2, wherein the human milk oligosaccharide is provided in a form of natural origin, and/or synthetic origin, and/or bacterial fermentation origin.
4. The nutrient composition of any one of items 1 to 3, wherein:
   the milk fat globule membrane is cow and/or sheep milk-origin phospholipid; and/or
   the milk fat globule membrane is provided in the form of an protein powder containing milk fat globule membrane, said protein powder containing milk fat globule membrane preferably having a milk fat globule membrane content of 6~25% by weight; and/or of a cow milk-origin phospholipid, said cow milk-origin phospholipid preferably having a milk fat globule membrane content of 9~60% by weight; and/or
   the milk fat globule membrane at least contains sphingophospholipid and phosphatidylcholine, and optionally further contains phosphatidylserine and/or phosphatidylethanolamine, wherein the sphingophospholipid represents 10% by weight or more of the total milk fat globule membrane, and the phosphatidylcholine represents 15% by weight or more of the total milk fat globule membrane.
5. The nutrient composition of any one of items 1 to 4, wherein the choline and/or edible choline derivative is choline chloride and/or choline bitartrate.
6. The nutrient composition of any one of items 1 to 5, wherein:
   a mass ratio of the human milk oligosaccharide to the milk fat globule membrane is 1:0.01-500, preferably 1:0.02-100, preferably 1:0.05-50, preferably 1:0.1-10, preferably 1:0.15-6; and
   a mass ratio of the human milk oligosaccharide to the choline and/or edible choline derivative is 1:0.001-100, preferably 1:0.002-20, preferably 1:0.005-5, preferably 1:0.01-1, preferably 1:0.02-0.5, as a ratio of a mass of the human milk oligosaccharide to an equivalent choline total mass;
   preferably, a mass ratio of the human milk oligosaccharide to the milk fat globule membrane to the choline and/or edible choline derivative is 1:0.01-500:0.001-100, preferably 1:0.02-100:0.002-20, preferably 1:0.05-50:0.005-5, preferably 1:0.1-10:0.01-1, preferably 1:0.15-6:0.02-0.5, as a ratio of a mass of the human milk oligosaccharide to a mass of the milk fat globule membrane to the equivalent choline total mass.
7. A food comprising the nutrient composition of any one of items 1 to 6.
8. The food of item 7, wherein said food is in the form of powder or liquid.
9. The food of any one of items 7 to 8, which is a formula food for infants and young children, for example a formula food for infants, a formula food for more grown-up infants, a formula food for young children, for example a formula milk powder for infants and young children, for example a formula milk powder for infants, and a formula milk powder for young children; a complementary food for infants; nutritional or dietary supplements; or a formulated milk powder for pregnant women.
10. The food of any one of items 7 to 9, wherein the amount of said nutrient composition added, relative to the total weight of said food, is such that:
   the human milk oligosaccharide is at a content by weight of at least 0.01%, preferably at least 0.05%, preferably at least 0.1% and at most 10.0%, preferably at most 5.0%, preferably at most 1.0%,
   the milk fat globule membrane is at a content by weight of at least 0.01%, preferably at least 0.05%, preferably at least 0.1% and at most 5.0%, preferably at most 1.0%, preferably at most 0.6%, and
   the choline or edible choline is at a content by weight of at least 0.01%, preferably at least 0.05%, preferably at least 0.1% and at most 3.0%, preferably at most 1.0%, preferably at most 0.3%, wherein a mass of the edible choline is converted into a mass of choline.
11. Use of the nutrient composition of any one of items 1 to 6 or the food of any one of items 7 to 10 for non-curing purposes of improving the brain development and intelligence of infants and young children, especially promoting neurodevelopment such as neuron maturation, synaptogenesis and myelination, in particular promoting the the oligodendrocyte precursor cell proliferation, maturation, and differentiation into oligodendrocytes and/or myelination of oligodendrocytes.

### Detailed Description of the Invention

Unless otherwise specified, the scientific and technical terms in the present description have the same meaning as that generally understood by those skilled in the art, . However, in case of conflict, the definition in the present description should prevail.

As used herein, the following terms have the meaning below.

The term "infant" refers to a 0~6 month-aged person.

The term "more grown-up infant" refers to a 6~12 month-aged person.

The term "young children" refers to a 12~36 month-aged person.

The term "infant and young child" refers to a 0-36 month-aged person.

The term "formula food for infants and young children" used herein encompasses formula food for infants, formula food for more grown-up infants, and formula food for young children. Generally, formula food for infants serves as an alternative for human milk from a birth of an infant, formula food for more grown-up infants serves as an alternative for human milk from 6 to 12 months after the birth of an infant, and formula food for young children serves as an alternative for human milk from 12 to 36 months after the birth of an infant.

The term "formula food for infants" refers to a liquid-state or powder-like product produced, processed and made only via physical methods, with milk and lactoprotein (milk protein) products or soybean and soybean protein products as major raw materials, and with addition of a proper amount of vitamin(s), minerals, and/or other constituent(s). It is suitable for ordinary infants, with its energy and nutrients being able to meet an ordinary (normal) nutritional requirement for a 0-6 month-aged infant.

The term "formula food for more grown-up infants" refers to a liquid-state or powder-like product produced, processed and made only via physical methods, with milk and lactoprotein (milk protein) products or soybean and soybean protein products as major raw materials, and with addition of a proper amount of vitamin(s), minerals, and/or other constituent(s). It is suitable for more grown-up infants, with its energy and nutrients being able to meet part of a nutritional requirement for a 6-12 month-age ordinary (normal) more grown-up infants.

The term "formula food for young children" refers to a liquid-state or powder-like product produced, processed and made only via physical methods, with milk and lactoprotein (milk protein) products or soybean and soybean protein products as major raw materials, and with addition of a proper amount of vitamin(s), minerals, and/or other constituent(s). It is suitable for young children, with its energy and nutrients being able to meet part of a nutritional requirement for a 12-36 month-age ordinary (normal) young children.

The term "human milk" should be understood as mother's human milk or colostrum.

The term "infants or young children fed entirely with human milk" has common meaning and refers to an infant whose majority of nutrition and/or energy originates from human milk.

The term "infants/more grown-up infants/young children fed mainly with formula food for infants and young children" has common meaning and refers to an infant or young children whose nutritional source of nutrition and/or energy mainly originates from formula food for infants and young children and milk or growing up milk for more grown-up infants, which are produced, processed and made only via physical methods. The term "mainly / majority of' refers to at least 50%, for example at least 75% of those nutrition and/or energy.

In addition, in the context of the present invention, the term "contain(s) / containing / contained", "include(s) / including / included" or "comprise(s) / comprising / comprised" do not exclude other elements possible. The composition of the present invention (including embodiments described herein) may comprise, consist of, or consist essentially of the following elements: the basic elements and necessary restraints of the present invention described herein, and any other or optional constituents, components or restraints described herein or as may be desired.

Individuals described in the present invention applies to ordinary human beings, and may be infants and/or more grown-up infants, and/or young children, and/or children, and/or adolescents (the youth/young people), and/or middle-aged people, and/or old people, more preferably, human infants and young children fed with formula food.

All percentages are percentages by weight, unless indicated otherwise.

The present incention will now be described in greater detail. It should be noted that various aspects, features, embodiments, examples and anvantages thereof described in the present application are compatible and/or can be combined together.

The present invention relates to a composition for improving the brain development and intelligence of infants and young children, especially promoting neurodevelopment such as neuron maturation, synaptogenesis and myelination, a food containing the nutrient composition, as well as a use of said nutrient composition for non-curing purposes of improving the brain development and intelligence of infants and young children, especially promoting neurodevelopment such as neuron maturation, synaptogenesis and myelination, in particular promoting oligodendrocyte precursor cell proliferation, maturation, and differentiation into oligodendrocytes and/or myelination of oligodendrocytes.

The present invention will be described in detail hereinbelow.

### Nutrient Composition

In one aspect, the present invention provides a nutrient composition comprising:
- human milk oligosaccharide (HMO), preferably neurtral fucosylated human milk oligosaccharide (HMO), preferably one or more selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), preferably 2'-fucosyl lactose;
- milk fat globule membrane; and
- choline and/or edible choline derivative.

Human milk oligosaccharide HMO is a general term for oligosaccharides with a degree of polymerization of ≥3 naturally present in human milk . It is based on a lactose molecule with its ends being modified with 5 classes of monoers, i.e. glucose (Glc), galactose (Gal), N-acetyl glucasamine (GlcNAc), fucose (Fuc), and N-acetyl neuraminic acid (Neu5Ac). HMO comprises 3 to 32 monosaccharides per molecule, these monosaccharides being connected by various glycosidic bonds, constituting the versatility and complexity of HMO. Based on its core structure, HMO may be classified into three types, i.e. neutral fucosylated HMO (containing fucose at its end), neutral non-fucosylated HMO (containing N-acetyl glucasamine at its end), and acidic or salylated HMO (containing sialic acid at its end), representing a proportion of 35-50%, 42-55%, and 12-14% in human milk oligosaccharide, respectively.

2'-fucosyl lactose (2'-FL) is a neutral trisaccharide consisting of an L-fucose, a D-galactose and a D-glucose unit, wherein the monosaccharide L-fucose is connected to the disaccharide D-lactose via an α(x→2) bond. It has a molecular fomula of C₁₈H₃₁O₁₅, a molecular weight of 488.439 g/mol, and a molecular structure of

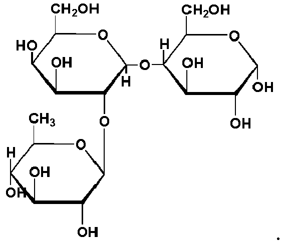

3'-fucosyl lactose (3'-FL) is a neutral trisaccharide consisting of an L-fucose, a D-galactose and a D-glucose unit, wherein the monosaccharide L-fucose is connected to the disaccharide D-glucose via an α(1→3) bond. It has a molecular formula of C₁₈H₃₂O₁₅, a molecular weight of 488.44 g/mol, and a molecular structure of

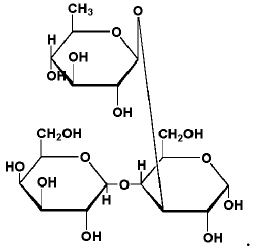

Lacto-N-fucopentaose I (LNFP I) is a neutral pentaose consisting of an L-fucose, a D-glucose, 2 molecules of D-galactose, and an N-acetyl glucasamine unit, wherein the monosaccharide L-fucose is connected to the D-glucose via an α(1→3) bond. It has a molecular formula of C₃₂H₅₅NO₂₅, a molecular weight of 853.77 g/mol, and a molecular structure of

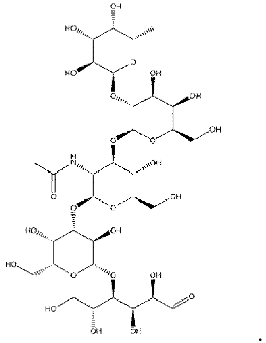

Lacto-N-difucohexaose I (LNDFH I) is a neutral hexaose consisting of 2 molecules of L-fucose, a D-glucose, a D-galactose, and an N-acetyl glucasamine unit, wherein the monosaccharide L-fucose is connected to the D-galactose and the N-acetyl glucasamine via an α(1→2) bond and an α(1→4) bond, respectively. It has a molecular formula of C₃₈H₆₅NO₂₉, a molecular weight of 999.91 g/mol, and a molecular structure of

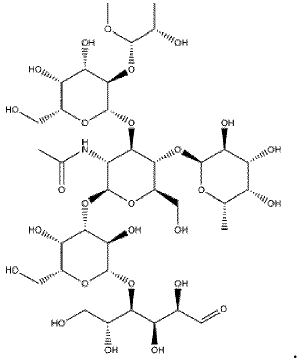

Lacto-N-difucohexaose II (LNDFH II) is a neutral hexaose consisting of 2 molecules of L-fucose, a D-glucose, a D-galactose, and an N-acetyl glucasamine unit, wherein the monosaccharide L-fucose is connected to the D-glucose and the N-acetyl glucasamine via an α(1→3) bond and an α(1→4) bond, respectively. It has a molecular formula of C₃₈H₆₅NO₂₉, a molecular weight of 999.91 g/mol, and a molecular structure of

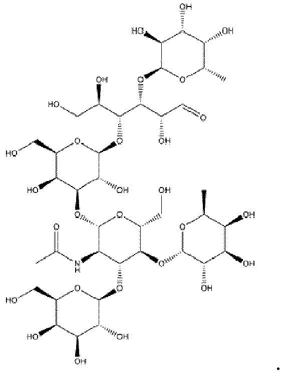

Sphingophospholipid, a sphingolipid constituted by attaching choline phosphate (or ethanolamine phosphate) on a C-1 hydroxyl of ceramide, has a molecular structure of:

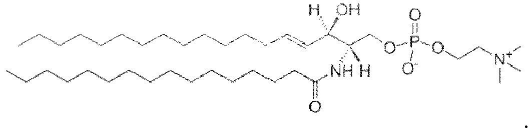

Phosphatidylethanolamine, 1,2-dipalmitoyl-SN-glycerol-3-phosphoryl ethanolamine, has a molecular structure of:

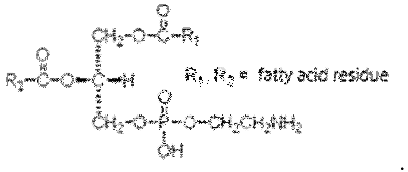

Choline is a tetravalent basic group with positive charge. It is a component of all biomembranes, and a precursor of acetylcholine in cholinergic neurons. Choline has a chemical formula of C₅H₁₄ON⁺.

Herein, an editable choline derivative refers to a substance which can be decomposed into choline in human body or animal body and has no biotoxicity. It complies with relevant national standards for food.

The inventors have surprisingly discovered that when human milk oligosaccharide (HMO) (preferably a neurtral fucosylated human milk oligosaccharide selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), preferably 2'-fucosyl lactose (2'-FL)), milk fat globule membrane, and choline and/or edible choline derivative are used in combination, they can synergistically promote neurodevelopment such as neuron maturation, synaptogenesis and myelination, in particular they can synergistically promote OPC proliferation, maturation and differentiation into mature OL, and/or OL myelination characteristics.

In one embodiment, said nutrient composition consists of: the human milk oligosaccharide (HMO), preferably neurtral fucosylated human milk oligosaccharide (HMO), preferably one or more selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), preferably 2'-fucosyl lactose (2'-FL); the milk fat globule membrane; and the choline and/or edible choline derivative.

In one embodiment, said human milk oligosaccharide is a neurtral fucosylated human milk oligosaccharide (HMO), preferably one or more neurtral fucosylated human milk oligosaccharides (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II).

In one embodiment, said human milk oligosaccharide is 2'-fucosyl lactose (2'-FL).

In one embodiment, said human milk oligosaccharide (HMO) (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), for example 2'-fucosyl lactose (2'-FL)) can be provided in the form of the following origins: natural origin, and/or synthetic origin, and/or bacterial fermentation origin. For example, for 2'-fucosyl lactose, 2'-fucosyl lactose can generally be a content of 60-99.9% in various sources thereof.

In one embodiment, the milk fat globule membrane may be cow and/or sheep milk-origin phospholipid.

In one embodiment, the milk fat globule membrane may be provided in the form of, or may originate from: protein powder containing milk fat globule membrane, and/or cow milk-origin phospholipid. The protein powder containing milk fat globule membrane may commonly have a milk fat globule membrane content of 6~25% by weight, for example 7~15% by weight. The cow milk-origin phospholipid may commonly have a milk fat globule membrane content of 9~60% by weight. The milk fat globule membrane at least contains sphingophospholipid and phosphatidylcholine, and optionally further contains phosphatidylserine and/or phosphatidylethanolamine, wherein the sphingophospholipid represents a ratio of 10% by weight or more of the total milk fat globule membrane, and the phosphatidylcholine represents a ratio of 15% by weight or more of the total milk fat globule membrane. In one embodiment, the phospholipid constituents in the milk fat globule membrane represent the following ratio of the total phospholipid: sphingophospholipid (SM) 15-36 %, phosphatidylcholine (PC) 20-40 %, phosphatidylethanolamine (PE) 20-38 %, phosphatidylserine (PS) 5-18 %, and phosphatidylinositol (PI) 3-11 %.

In one embodiment, the edible choline derivative is choline chloride or choline bitartrate. The choline chloride described herein complies with the requirements of GB 1903.36 for food nutrition enhancing agent choline chloride, and the choline bitartrate described herein complies with the requirements of GB 1903.54-2021 for food nutrition enhancing agent choline bitartrate.

In one embodiment, the mass ratio of the human milk oligosaccharide (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), lacto-N-difucohexaose II (LNDFH II), for example 2'-fucosyl lactose (2'-FL)) to the milk fat globule membrane in said nutrient composition may be 1:0.01-500, preferably 1:0.02-100, preferably 1:0.05-50, preferably 1:0.1-10, preferably 1:0.15-6, for example may be 1:0.01, 1:0.015, 1:0.02, 1:0.025, 1:0.03, 1:0.04, 1:0.05, 1:0.06, 1:0.07, 1:0.08, 1:0.09, 1:0.1, 1:0.15, 1:0.2, 1:0.25, 1:0.3, 1:0.4, 1:0.5, 1:0.6, 1:0.7, 1:0.8, 1:0.9, 1:1.0, 1:1.5, 1: 2.0, 1:2.5, 1:3.0, 1:3.5, 1:4.0, 1:4.5, 1:5.0, 1:5.5, 1:6.0, 1:7.0, 1:8.0, 1:9.0, 1:10.0, 1:11.0, 1:12.0, 1:13.0, 1:14.0, 1:15.0, 1:16.0, 1:17.0, 1:18.0, 1:19.0, 1:20.0, 1:21.0, 1:22.0, 1:23.0, 1:24.0, 1:25.0, 1:26.0, 1:27.0, 1:28.0, 1:29.0, 1:30.0, 1:31.0, 1:32.0, 1:33.0, 1:34.0, 1:35.0, 1:36.0, 1:37.0, 1:38.0, 1:39.0, 1:40.0, 1:41.0, 1:42.0, 1:43.0, 1:44.0, 1:45.0, 1:46.0, 1:47.0, 1:48.0, 1:49.0, 1:50.0, 1:51.0, 1:52.0, 1:53.0, 1:54.0, 1:55.0, 1:56.0, 1:57.0, 1:58.0, 1:59.0, 1:60.0, 1:61.0, 1:62.0, 1:63.0, 1:64.0, 1:65.0, 1:66.0, 1:67.0, 1:68.0, 1:69.0, 1:70.0, 1:71.0, 1:72.0, 1:73.0, 1:74.0, 1:75.0, 1:76.0, 1:77.0, 1:78.0, 1:79.0, 1:80.0, 1:81.0, 1:82.0, 1:83.0, 1:84.0, 1:85.0, 1:86.0, 1:87.0, 1:88.0, 1:89.0, 1:90.0, 1:91.0, 1:92.0, 1:93.0, 1:94.0, 1:95.0, 1:96.0, 1:97.0, 1:98.0, 1:99.0, 1:100, 1:110, 1:120, 1:130, 1:140, 1:150, 1:160, 1:170, 1:180, 1:190, 1:200, 1:210, 1:220, 1:230, 1:240, 1:250, 1:260, 1:270, 1:280, 1:290, 1:300, 1:310, 1:320, 1:330, 1:340, 1:350, 1:360, 1:370, 1:380, 1:390, 1:400, 1:410, 1:420, 1:430, 1:440, 1:450, 1:460, 1:470, 1:480, 1:490, 1:500, or a range defined by any two thereof and any value and subrange contained within the range.

In one embodiment, the mass ratio of the human milk oligosaccharide (for example neutral fucosylated human milk oligosaccharide(HMO) selected from the group consisting of 2'-fucosyl lactose(2'-FL), 3'-fucosyl lactose(3'-FL), lacto-N-fucopentaose I (LNFP I), lacto-N-difucohexaose I (LNDFH I), lacto-N-difucohexaose II (LNDFH II), for example 2'-fucosyl lactose(2'-FL)) to the choline or edible choline in said nutrient composition may be 1:0.001-100, preferably 1:0.002-20, preferably 1:0.005-5, preferably 1:0.01-1, preferably 1:0.02-0.5, for example may be 1:0.001, 1:0.002, 1:0.003, 1:0.004, 1:0.005, 1:0.006, 1:0.007, 1:0.008, 1:0.009, 1:0.01, 1:0.02, 1:0.03, 1:0.04, 1:0.05, 1:0.06, 1:0.07, 1:0.08, 1:0.09, 1:0.1, 1:0.2, 1:0.3, 1:0.4, 1:0.5, 1:0.6, 1:0.7, 1:0.8, 1:0.9, 1:1.0, 1:1.5, 1: 2.0, 1:2.5, 1:3.0, 1:3.5, 1:4.0, 1:4.5, 1:5.0, 1:6.0, 1:7.0, 1:8.0, 1:9.0, 1:10.0, 1:11.0, 1:12.0, 1:13.0, 1:14.0, 1:15.0, 1:16.0, 1:17.0, 1:18.0, 1:19.0, 1:20.0, 1:21.0, 1:22.0, 1:23.0, 1:24.0, 1:25.0, 1:26.0, 1:27.0, 1:28.0, 1:29.0, 1:30.0, 1:31.0, 1:32.0, 1:33.0, 1:34.0, 1:35.0, 1:36.0, 1:37.0, 1:38.0, 1:39.0, 1:40.0, 1:41.0, 1:42.0, 1:43.0, 1:44.0, 1:45.0, 1:46.0, 1:47.0, 1:48.0, 1:49.0, 1:50.0, 1:51.0, 1:52.0, 1:53.0, 1:54.0, 1:55.0, 1:56.0, 1:57.0, 1:58.0, 1:59.0, 1:60.0, 1:61.0, 1:62.0, 1:63.0, 1:64.0, 1:65.0, 1:66.0, 1:67.0, 1:68.0, 1:69.0, 1:70.0, 1:71.0, 1:72.0, 1:73.0, 1:74.0, 1:75.0, 1:76.0, 1:77.0, 1:78.0, 1:79.0, 1:80.0, 1:81.0, 1:82.0, 1:83.0, 1:84.0, 1:85.0, 1:86.0, 1:87.0, 1:88.0, 1:89.0, 1:90.0, 1:91.0, 1:92.0, 1:93.0, 1:94.0, 1:95.0, 1:96.0, 1:97.0, 1:98.0, 1:99.0, 1:100, or a range defined by any two thereof and any value and subrange contained within the range, as a ratio of a mass of the human milk oligosaccharide to an equivalent choline total mass.

In one embodiment, the mass ratio of the human milk oligosaccharide (for example neutral fucosylated human milk oligosaccharide (HMO) selected from the group consisting of 2'-fucosyl lactose(2'-FL), 3'-fucosyl lactose(3'-FL), lacto-N-fucopentaose I (LNFP I), lacto-N-difucohexaose I (LNDFH I), and lacto-N-difucohexaose II (LNDFH II), for example 2'-fucosyl lactose(2'-FL)) to the milk fat globule membrane to the choline and/or edible choline derivative in said nutrient composision may be 1:0.01-500:0.001-100, preferably 1:0.02-100:0.002-20, preferably 1:0.05-50:0.005-5, preferably 1:0.1-10:0.01-1, preferably 1:0.15-6:0.02-0.5, or a mass ratio obtained by any combination of the ratios described for the mass ratio of the human milk oligosaccharide (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), for example 2'-fucosyl lactose (2'-FL)) to the milk fat globule membrane with the ratios described for the mass ratio of the human milk oligosaccharide (for example neutral fucosylated human milk oligosaccharide(HMO) selected from the group consisting of 2'-fucosyl lactose(2'-FL), 3'-fucosyl lactose(3'-FL), lacto-N-fucopentaose I (LNFP I), lacto-N-difucohexaose I (LNDFH I), and lacto-N-difucohexaose II (LNDFH II), for example 2'-fucosyl lactose(2'-FL)) to the choline or edible choline (converted into the mass of choline for calculation), as a mass ratio of the human milk oligosaccharide to the milk fat globule membrane to equivalent choline.

Herein, when a mass ratio of choline and/or edible choline derivative to other substance(s), and a content by mass of choline and/or edible choline in a composition are mentioned, the edible choline derivative has been converted into a mass of choline for calculation, unless otherwise indicated; that is to say, when there is only choline and no edible choline derivatives, said mass ratio and said content by mass refer to a mass ratio of choline to other substance(s), and a percentage of choline mass relative to a total mass of the composition, respectively; when there is only an edible choline derivative and no choline, said mass ratio and content by mass refer to a mass ratio of the mass of choline converted from the edible choline derivative or corresponding to the edible choline derivative to the mass of other substance(s), and a percentage of the mass of choline converted from the edible choline derivative or corresponding to the edible choline derivative, relative to the total mass of the composition, respectively; when there are choline and edible choline derivatives, said mass ratio and content by mass refer to a ratio of a sum of the mass of choline and the mass of choline converted from the edible choline derivative or corresponding to the edible choline derivative to the mass of other substances, and a percentage of the sum relative to the total mass of the composition, respectively.

When the mass ratio of the human milk oligosaccharide (for example neurtral fucosylated human milk oligosaccharide (HMO) selected from the group consisting of 2'-fucosyl lactose (2'-FL), 3'-fucosyl lactose (3'-FL), lacto-N-fucopentaose I (LNFP I), lacto-N-difucohexaose I (LNDFH I), lacto-N-difucohexaose II (LNDFH II), for example 2'-fucosyl lactose (2'-FL)), the milk fat globule membrane, and the choline or edible choline (converted into the mass of choline for calculation) in the nutrient composition falls within the above-mentioned ranges, more significant promotion in terms of neurodevelopment such as neuron maturation, synaptogenesis and myelination and the like, in particular promotion in terms of OPC proliferation, maturation and differentiation into mature OL and/or of OL myelination characteristics, can be realized, and there is more significant synergistic action among various components in terms of these aspects.

### Food

In another aspect, the present invention also relates to a food containing said nutrient composition.

The food of the present invention may be in a form of powder, and it may also be in a form of liquid.

The food of the present invention may be a formula food for infants and young children (e.g. a formula food for infants, a formula food for more grown-up infants, and a formula food for young children), for example a formula milk powder for infants and young children (e.g. a formula milk powder for infants, and a formula milk powder for young children), a complementary food for infants, nutritional or dietary supplements, or a formulated milk powder for pregnant women.

In one embodiment, said nutrient composition is added in an amount such that the human milk oligosaccharide (e.g. neurtral fucosylated human milk oligosaccharide (HMO) selected from the group consisting of 2'-fucosyl lactose (2'-FL),3'-fucosyl lactose (3'-FL), lacto-N-fucopentaose I (LNFP I), lacto-N-difucohexaose I (LNDFH I), lacto-N-difucohexaose II (LNDFH II), for example 2'-fucosyl lactose (2'-FL)) is at a content by weight of at least 0.01%, preferably at least 0.05%, preferably at least 0.1% and at most 10.0%, preferably at most 5.0%, preferably at most 1.0%, relative to the total weight of said food. For example, 2'-fucosyl lactose (2'-FL) may be at a content by weight, relative to the total weight of said food, of 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10.0%, or a range defined by any two thereof and any value and subrange contained within the range.

In one embodiment, said nutrient composition is added in an amount such that the milk fat globule membrane is at a content by weight, relative to the total weight of said food, of at least 0.01%, preferably at least 0.05%, preferably at least 0.1% and at most 5.0%, preferably at most 1.0%, preferably at most 0.6%. For example, the milk fat globule membrane may be at a content by weight, relative to the total weight of said food, of 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, or a range defined by any two thereof and any value and subrange contained within the range.

In one embodiment, said nutrient composition is added in an amount such that the choline and/or edible choline derivative (converted into the mass of choline for calculation) is at a content by weight of at least 0.01%, preferably at least 0.05%, preferably at least 0.1% and at most 3.0%, preferably at most 1.0%, preferably at most 0.3%, relative to the total weight of said food. For example, choline may be at a content by weight of 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.10%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.41%, 0.42%, 0.43%, 0.44%, 0.45%, 0.46%, 0.47%, 0.48%, 0.49%, 0.50%, 0.51%, 0.52%, 0.53%, 0.54%, 0.55%, 0.56%, 0.57%, 0.58%, 0.59%, 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, or a range defined by any two thereof and any value and subrange contained within the range, relative to the total weight of said food. As described above, in terms of a content by mass (weight) of the choline and/or edible choline derivative, the edible choline derivative has been converted into a mass of choline for calculation; that is to say, when there is only choline and no edible choline derivatives, said content by mass refers to a content by mass of choline to a total mass of the composition; when there is only an edible choline derivative and no choline, said content by mass refers to a percentage of the mass of choline converted from the edible choline derivative or corresponding to the edible choline derivative, relative to the total mass of the composition; when there are choline and edible choline derivatives, said content by mass refers to a percentage of a sum of the mass of choline and the mass of choline converted from the edible choline derivative or corresponding to the edible choline derivative relative to the total mass of the composition.

In addition the constituents described above for said nutrient composition, said food may have other constituents, for example other proteins, carbohydrates, fat, vitamins, minerals and the like, which are commonly contained in formula food, for example formula food like milk powder for infants and young children.

### Use

In another aspect, the present invention relates to use of the nutrient composition or food described above for non-curing purposes (i.e., non-medical purpose) of improving the brain development and intelligence of infants and young children, especially promoting neurodevelopment (for example neuron maturation, synaptogenesis and myelination, for example promoting OPC proliferation, maturation, and differentiation into mature oligodendrocytes (OLs) and/or myelination of oligodendrocytes (OLs)).

### Example

The present invention will be described in greater detail hereinbelow in combination with the examples, such that the objects, technical solutions and advantages of the present invention will be more apparent. It should be understood that the specific examples described herein is only for explanation of the present invention rather than limination of the present invention. The reagents, methods and devices employed in the present invention are ordinary reagents, methods and devices in the art, unless specifically indicated.

### Raw materials

The following raw materials are used in the example section which follows, unless indicated otherwise.
2'-fucosyl lactose (2'-FL): GlyCare^{™} 2'-FL 9000, 2'-fucosyl lactose content 96.0 % by mass
Milk fat globule membrane powder (phospholipid 12.3%): Tatua of New Zealand PLC1
Cholin: choline chloride from Beijing Jinkangpu Food Technology Co., Ltd, with a choline content of 72.5 % by mass.

In the following section, unless indicated otherwise, choline chloride is abbreviated as choline, and when a part by mass and ratio of the constituents in the nutrient composition are mentioned, the part by mass refers to the part by mass of 2'-fucosyl lactose (2'-FL), milk fat globule membrane, and choline, which are active constituents, and the ratio refers to the mass ratio among 2'-fucosyl lactose (2'-FL), milk fat globule membrane, and choline, which are active constituents, i.e. wherein the mass of choline chloride has been converted to the mass of cholin for calculation.

Several nutrient compositions are formulated by mixing the raw materials of 2'-fucosyl lactose (2'-FL), milk fat globule membrane, and choline according to the ratios shown in the following examples, the composition of which are shown in table 1.

**Table 1. Composition of the nutrient compositions**

| No. | Part by mass of 2'-FL | Part by mass of milk fat globule membrane | Part by mass of choline |
|---|---|---|---|
| Example 1 | 1 | 2.75 | 0.325 |
| Example 2 | 1 | 0.275 | 0.0325 |
| Example 3 | 1 | 0.275 | 0.325 |
| Comparative Example 1 | 0 | 0 | 1 |
| Comparative Example 2 | 1 | 0 | 0 |
| Comparative Example 3 | 0 | 1 | 0 |
| Comparative Example 4 | 1 | 0.275 | 0 |
| Comparative Example 5 | 1 | 0 | 0.325 |
| Comparative Example 6 | 0 | 0.275 | 0.325 |

### Cell Experiment Example

Current studies on brain development are limited to studies on passive memory using animal experiments. There is no discussion on the development of neuron cells, nor is there exploration on the effect of specific dosage of the composition on early brain development. The present invention uses an *in vitro* model of culture of primary cell containing neurons and OPC to evaluate the effect of a nutrient mixture on myelination and neurons. Through experimental research on the effects of components containing 2'-FL, milk fat globle membrane and choline in varying proportions on the brain neurve cell development, this method systematically and intuitively evaluated the effects on early brain development, which is of profound significance in terms of guiding the application of the composition.

### 1. Materials and methods

### 1.1 Apparatus and reagent consumables

Cell incubator; Laser confocal fluorescence microscope; Centrifuge; Electronic balance; Vortexer, water bath, tissue homogenizer.

Nerve cell culture medium; A2B5, MAG, MBP and other antibodies.

### 1.2 Experimental Method

### 1.2.1 Acquisition of nerve primary cells

All experiments were approved by ethics, and the primary mixed culture of neurons and OL were carried out according to the following steps. Briefly, the forebrain of newborn rats and mice were dissociated with trypsin (Trypsin EDTA IX, PAN BIOTECH) at 37 °C for 20 minutes. After addition of Dulbecco modified Eagle medium (DMEM, PAN BIOTECH) containing DNAase I Grade II (0.1 mg/ml, PAN BIOTECH) and 10% fetal bovine serum (FCS, GIBCO), the reaction was stopped. The cells were mechanically separated three times through a 10 ml pipette, and then centrifuged at 4 °C under 515g for 10 minutes.

Living cells were inoculated in 96-well plates (20000/well), which were pre-coated with poly-L-lysine (BD Falcon) and laminin (Sigma). The culture medium consists of Neurobasal (GIBCO) added with 2% B27 (GIBCO), 2 mM L-glutamine (L Glu, PAN BIOTECH), 2% P/S solution (PAN BIOTECH), 1% FCS and 10 ng/ml platelet-derived growth factor (PDGF-AA, PAN BIOTECH). The 96-well plates are stored in a humidified incubator, at a temperature of 37 °C and with an environment of air (95%) - CO₂ (5%).

### 1.2.2 Nerve cell culture

A same number of cells were placed on 48-well plates, and then incubated for 12, 18 or 30 DIVs. Half of the medium were replaced every other day. A mixture or separate nutrition were added to the fresh medium (the nutrient composition prepared above, containing 2'-FL, choline and/or milk fat global membrane).

### 1.2.3 Immunohistochemical experiment

After 12, 18 and 30 DIVs, the cells were fixed with a cold mixture of 95% ethanol and acetic acid (5%) for 5 minutes. The non-specific sites were then blocked for 15 minutes at room temperature with a phosphate buffered saline (PBS) solution containing 0.1% saponin (Sigma) and 1% FCS (GIBCO).

At 12 DIVs, nerve cells were co-incubated with mouse monoclonal antibody A2B5 (dilution: 1/200, Millipore, MAB312RX) at room temperature for 2h, and then co-incubated with neurofilament antibody (dilution: 1/500, Sigma, N4142) at room temperature for 2h. Finally, a secondary goat anti-rabbit antibody (diluted 1/400, SIGMA, SAB460084) was used to co-incubate at room temperature for 1 h.

At 18 DIVs, nerve cells were co-incubated with mouse monoclonal antibody MAG (dilution: 1/400, Millipore, MAB1567) and neurofilament protein antibody (dilution: 1/500, Sigma, N4142) for 2h. Then a secondary goat anti-rabbit antibody (dilution: 1/400, Sigma, SAB4600042) and a secondary goat anti-rabbit antibody (dilution: 1/400, SIGMA, SAB4600084) were used to co-incubate at room temperature for 1h.

At 30 DIVs, nerve cells were co-incubated with mouse monoclonal antibody MBP (dilution: 1/1000, Novus, NBP1-05204) and neurofilament protein antibody (dilution: 1/500, Sigma, N4142) for 2 hours. Then a secondary goat anti-mouse antibody (dilution: 1/800, Sigma, SAB4600042) and a secondary goat anti-rabbit antibody (dilution: 1/400, SIGMA, SAB4600084) were used to co-incubate at room temperature for 1h.

### 1.2.4 Microscrope imaging

ImageXpress equipped with LED lights (360/480/565 for excitation and 460/535/620 for emission) is used for 20x amplification. All images are acquired using the same settings.

Under the condition of 12 DIVs, the number of OPC was calculated by quantifying the number of A2B5 expressing cells, and the result was expressed as the average number of A2B5 expressing cells per image and per well.

The differentiation of OPC into OL was evaluated by counting the number of MAG positive cells in cell culture. The results were expressed as the average number of cells per image and per well. The morphological maturity of MAG positive cells were assessed by measuring the average surface area of MAG positive cells in 18 DIV wells (µM/image/well).

At 30 DIVs, the maturity of OL was estimated by calculating the number of MBP positive cells (average number of cells per imge, per well) and the average surface area of MBP positive cells at 30 DIVs (µM, per image, per well).

### 1.3 cell experiment grouping

Cells were inoculated on a 96-well plate and cultured for a certain period of time. Half of the culture medium was replaced every other day. Varying concentrations of mixed or separate 2'-FL mixtures were added to the special culture medium for fresh primary cells (added on day 12, 18 and 30, respectively). 6 replicas for each sample. Oleoxime (300 nM, proved to accelerate OL maturation and myelination in vitro and in vivo) was taken as the positive control. The blank control group, positive control group and sample intervention group were compared, respectively. The effects of milk fat global membrane, 2'-FL and choline, alone or in mixture, on OPC population, OL maturation and differentiation, myelin formation and neurite growth was determined by immunohistochemistry (MBP, NF, A2B5). See Table 2 below for specific experimental dosage.

**Table 2. Dosage for Cell Experiments**

| Cell Experiment Example | Corresponding Example | Dosage Group | 2'-FL (mg/ml) | milk fat globule membrane (mg/ml) | Choline (mg/ml) |
|---|---|---|---|---|---|
| 1 | Control | blank control group | | | |
| 2 | positive control | positive control group Olesoxime | | | |
| 3 | Comparative Example 2 | 2'-FL (low dosage) | 0.2 | | |
| 4 | Comparative Example 2 | 2'-FL (medium dosage) | 2 | | |
| 5 | Comparative Example 2 | 2'-FL (high dosage) | 20 | | |
| 6 | Comparative Example 3 | milk fat globule membrane (low dosage) | | 0.55 | |
| 7 | Comparative Example 3 | milk fat globule membrane (medium dosage) | | 5.5 | |
| 8 | Comparative Example 3 | milk fat globule membrane (high dosage) | | 55 | |
| 9 | Comparative Example 1 | choline (low dosage) | | | 0.065 |
| 10 | Comparative Example 1 | choline (medium dosage) | | | 0.65 |
| 11 | Comparative Example 1 | choline (high dosage) | | | 6.5 |
| 12 | Comparative Example 4 | 2'-FL+milk fat globule membrane | 2 | 0.55 | |
| 13 | Comparative Example 5 | 2'-FL+choline | 2 | | 0.65 |
| 14 | Comparative Example 6 | milk fat globule membrane+choline | | 0.55 | 0.65 |
| 15 | Example 1 | 2'-FL (low dosage)+milk fat globule membrane (low dosage)+choline (low dosage) | 0.2 | 0.55 | 0.065 |
| 16 | Example 1 | 2'-FL (medium dosage)+milk fat globule membrane (medium dosage)+choline (medium dosage) | 2 | 5.5 | 0.65 |
| 17 | Example 1 | 2'-FL (high dosage)+milk fat globule membrane (high dosage)+choline (high dosage) | 20 | 55 | 6.5 |
| 18 | Example 1 | 2'-FL (low to medium dosage)+milk fat globule membrane (low to medium dosage)+choline (low to medium dosage) | 1 | 2.75 | 0.325 |
| 19 | Example 1 | 2'-FL (medium to high dosage)+milk fat globule membrane (medium to high dosage)+choline (medium to high dosage) | 4 | 11 | 1.3 |
| 20 | Example 2 | 2'-FL+milk fat globule membrane+cholin e | 2 | 0.55 | 0.065 |
| 21 | Example 3 | 2'-FL+milk fat globule membrane+choline | 2 | 5.5 | 0.65 |
| 22 | Example 4 | 2'-FL+milk fat globule membrane+choline | 2 | 0.55 | 0.65 |

### 1.4 Statistics analysis

The results were expressed as mean ± standard error. SPSS software was used for T-test and one-way ANOVA test. When p<0.05, it was judged that there was a significant difference

### 2. Experimental Results

### 2.1 Effect of various samples on nerve cells

In order to measure the effect of mixed nutrition or single nutrition treatment on OPC, we evaluated the number of positive cells labeled A2B5 after 12 DIVs to estimate the number of OPCs. The average number of A2B5 positive cells per well measured is shown in Table 3.

**Table 3. Effect of different nutrient composition samples comprising 2'-FL, milk fat global membrane and/or choline on the number of A2B5 positive cells**

| Cell Example | Dosage Group | number of A2B5 positive cells (mean±standard deviation) |
|---|---|---|
| 1 | blank control group | 116.67 ±3.79 |
| 2 | positive control group Olesoxime | 171.33±3.51^{∗∗} |
| 3 | 2'-FL (low dosage) | 151.33±4.73^{∗∗} |
| 4 | 2'-FL (medium dosage) | 156.00 ±5.57^{∗∗} |
| 5 | 2'-FL (high dosage) | 135.00±6.08^{∗∗} |
| 6 | milk fat globule membrane (low dosage) | 168.00±5.29^{∗∗} |
| 7 | milk fat globule membrane (medium dosage) | 131,33±6.11^{∗∗} |
| 8 | milk fat globule membrane (high dosage) | 106.33±8.74^{∗} |
| 9 | choline (low dosage) | 167.33±3.51^{∗∗} |
| 10 | choline (medium dosage) | 225.67±5.03^{∗∗} |
| 11 | choline (high dosage) | 107.67±6.03^{∗} |
| 12 | 2'-FL+milk fat globule membrane | 140.67±11.354^{∗∗} |
| 13 | 2'-FL+choline | 138.50±10.046^{∗∗} |
| 14 | milk fat globule membrane+choline | 135.67±10.834^{∗∗} |
| 15 | 2'-FL (low dosage)+milk fat globule membrane (low dosage)+choline (low dosage) | 206.00±5.57^{∗∗} |
| 16 | 2'-FL (medium dosage)+milk fat globule membrane (medium dosage)+choline (medium dosage) | 283.67±5.13^{∗∗} |
| 17 | 2'-FL (high dosage)+milk fat globule membrane (high dosage)+choline (high dosage) | 297.00±5.29^{∗∗} |
| 18 | 2'-FL (low to medium dosage)+milk fat globule membrane (low to medium dosage)+choline (low to medium dosage) | 184.67±5.51^{∗∗} |
| 19 | 2'-FL (medium to high dosage)+milk fat globule membrane (medium to high dosage)+choline (medium to high dosage) | 115.33±5.51 |
| 20 | 2'-FL+milk fat globule membrane+choline | 214.50±9.65^{∗∗} |
| 21 | 2'-FL+milk fat globule membrane+choline | 240.19±7.78^{∗∗} |
| 22 | 2'-FL+milk fat globule membrane+choline | 248.11±8.16^{∗∗} |
| N.B.: | ^{∗}p<0.05, in comparison with the blank control group, | |
| | ^{∗∗}p<0.01, in comparison with the blank control group | |

The results of sample treatments showed that all of 2'-FL, milk fat globule membrane, and choline may increase the number of A2B5 positive cells, in comparison with the blank control group (Cell Experiment Example 1), which indicates that all of these three constituents help the proliferation of oligodendrocyte precursor cells.

However, the inventors discovered that when any two of the three components were used in combination, there was a strong antagonism.

In particular, it can be seen according to Cell Experiment Examples 12-14 that:
- In terms of the comstituents used and the amount of the constituents used, Cell Experiment Example 12 is equivalent to a combination of Cell Experiment Examples 4 and 6. Relative to the blank control group (Cell Experiment Example 1), Cell Experiment Examples 4 and 6 increased the number of A2B5 positive cells by 39.33 and 51.33 respectively, , while Cell Experiment Example 12 increased the number of A2B5 positive cells by 24, which is not only far less than the sum of the former two (90.66) (a difference from the sum of the former two of Δ1=66.66), but also less than either one of the former two.
- Cell Experiment Example 13 is equivalent to a combination of Cell Experiment Examples 4 and 10. Relative to the blank control group (Cell Experiment Example 1), Cell Experiment Examples 4 and 10 increased the number of A2B5 positive cells by 39.33 and 109 respectively, while Cell Experiment Example 13 increased the number of A2B5 positive cells by 21.83, which is not only far less than the sum of the former two (148.33) (a difference from the sum of the former two of Δ2=126.5), but also less than either one of the former two.
- Cell Experiment Example 14 is equivalent to a combination of Cell Experiment Examples 6 and 10. However, relative to the blank control group (Cell Experiment Example 1), Cell Experiment Examples 6 and 10 increased the number of A2B5 positive cells by 51.33 and 109 respectively, while Cell Experiment Example 14 increased the number of A2B5 positive cells by 19, which is not only far less than the sum of the former two (160.33) (a difference from the sum of the former two of Δ3=141.33), but also less than either one of the former two.

This indicates that when any two of 2'-FL, milk fat global membrane and choline are used in combination, there is a strong antagonism in terms of the number of A2B5 positive cells and thus the proliferation of oligodendrocyte precursor cells, and the effect of their use in combination is less than that of any one of the components when it is used alone.

Moreover, the inventors found that when the three of 2'-FL, milk fat global membrane and choline are used in combination, a significant reduction in inter-component antagonism, and a synergistic increase in the number of A2B5 positive cells and thus the proliferation of oligodendrocyte precursor cells are enabled.

In particular, in terms of the comstituents used and the amount of the constituents used, Cell Experiment Example 16 is equivalent to a combination of Cell Experiment Examples 7 and 13, or a combination of Cell Experiment Examples 4, 7 and 10. Relative to Cell Experiment Example 1, Cell Experiment Examples 4, 7, 10, 13 and 16 increased the number of A2B5 positive cells by 39.33, 14.66, 109, 21.83 and 167 respectively, the increment in the number of A2B5 positive cells in Cell Experiment Example 16 (167) is greater than a sum of the respective increments of Cell Experiment Examples 7 and 13 (36.49), and also greater than a sum of the respective increments of Cell Experiment Examples 4, 7 and 10 (162.99).

In addition, in terms of the comstituents used and the amount of the constituents used, Cell Experiment Example 17 is equivalent to the combination of Cell Experiment Examples 5, 8 and 11. Relative to Cell Experiment Example 1, Cell Experiment Examples 5, 8 and 11 increased the number of A2B5 positive cells by 18.33, -10.34 and -9 respectively, while Cell Experiment Example 17 increased the number of A2B5 positive cells by 180.33, which is greater than the sum of the former three (-1.01).

In addition, in terms of the comstituents used and the amount of the constituents used, Cell Experiment Example 20 is equivalent to a combination of Cell Experiment Examples 9 and 12, or a combination of Cell Experiment Examples 4, 6 and 9. Relative to Cell Experiment Example 1, Cell Experiment Examples 4, 6, 9, 12 and 20 increased the number of A2B5 positive cells by 39.33, 51.33, 50.66, 24 and 97.83 respectively, the increment realized in Cell Experiment Example 20 (97.83) is greater than a sum of respective increments of Cell Experiment Examples 9 and 12 (74.66), less than a sum of respective increments of Cell Experiment Examples 4, 6 and 9 (141.32) (a difference of Δ4=43.69, less than Δ1=66.66).

In addition, in terms of the comstituents used and the amount of the constituents used, Cell Experiment Example 21 is equivalent to the combination of Cell Experiment Examples 7 and 13, or the combination of Cell Experiment Examples 4, 7 and 10. Relative to Cell Experiment Example 1, Cell Experiment Examples 4, 7, 10, 13 and 21 increased the number of A2B5 positive cells by 39.33, 14.66, 109, 21.83 and 123.52 respectively, the increment realized in Cell Experiment Example 21 (123.52) is greater than a sum of respective increments of Cell Experiment Examples 7 and 13 (36.49), less than a sum of respective increments of Cell Experiment Examples 4, 7 and 10 (162.99) (a difference of Δ5=39.47, less than Δ2=126.5).

In addition, in terms of the comstituents used and the amount of the constituents used, Cell Experiment Example 22 is equivalent to a combination of Cell Experiment Examples 4 and 14, or a combination of Cell Experiment Examples 6 and 13, or a combination of Cell Experiment Examples 10 and 12, ro a combination of Cell Experiment Examples 4, 6 and 10. Relative to the blank control group (Cell Experiment Example 1),
- Cell Experiment Example 22 increased the number of A2B5 positive cells by 131.44,
- Cell Experiment Examples 4 and 14 increased the number of A2B5 positive cells by 39.33 and 19 respectively, with a sum of 58.33, less than the increment realized in Cell Experiment Example 22 (131.44),
- Cell Experiment Examples 6 and 13 increased the number of A2B5 positive cells by 51.33 and 21.83 respectively, with a sum of 73.16, less than the increment realized in Cell Experiment Example 22 (131.44),
- Cell Experiment Examples 10 and 12 increased the number of A2B5 positive cells by 109 and 24 respectively, with a sum of 133, slightly greater than the increment realized in Cell Experiment Example 22 (131.44), with a difference of Δ6=1.56, far less than Δ1=66.66,
- Cell Experiment Examples 4, 6 and 10 increased the number of A2B5 positive cells by 39.33, 51.33 and 109 respectively, with a sum of 199.66, greater than the Increment realized in Cell Experiment Example 22 (131.44), with a difference of Δ7=68.22, far less than Δ2=126.5 and Δ3=141.33.

Thus, it can be seen that the three of 2'-FL, milk fat global membrane and choline is used in combination, a significantly reduction in inter-component antagonism is enabled. There is a synergistic effect among the three components, which can synergistically increase the number of A2B5 positive cells and thus the proliferation of oligodendrocyte precursor cells.

### 2.2 The effect of various samples on OPC cell myelination

In order to measure the effect of mixed nutrition or single nutrition treatment on OPC cell myelination, we evaluated the number of positive cells labelled MAG after 18 DIVs. The measured average number of MAG positive cells per well is shown in Table 4.

**Table 4. Effect of different nutrient composition samples containing 2'-FL, milk fat global membrane and/or choline on the number of MAG positive cells**

| Cell Example | Dosage Group | number of MAG positive cells (mean±standard deviation) |
|---|---|---|
| 1 | blank control group | 40.67±4.04 |
| 2 | positive control group Olesoxime | 87.00±4.36^{∗∗} |
| 3 | 2'-FL (low dosage) | 54.00±6.08^{∗∗} |
| 4 | 2'-FL (medium dosage) | 76.33±4.51^{∗∗} |
| 5 | 2'-FL (high dosage) | 62.00±4.00^{∗∗} |
| 6 | milk fat globule membrane (low dosage) | 70.33±4.04^{∗∗} |
| 7 | milk fat globule membrane (medium dosage) | 58.67±3.51^{∗∗} |
| 8 | milk fat globule membrane (high dosage) | 42.33±3.21 |
| 9 | choline (low dosage) | 66.33±6.03^{∗∗} |
| 10 | choline (medium dosage) | 94.00±4.58^{∗∗} |
| 11 | choline (high dosage) | 81.33±3.21^{∗∗} |
| 12 | 2'-FL+milk fat globule membrane | 66.50±6.662^{∗∗} |
| 13 | 2'-FL+choline | 65.17±6.467^{∗∗} |
| 14 | milk fat globule membrane+choline | 62.50±6.536^{∗∗} |
| 15 | 2'-FL (low dosage)+milk fat globule membrane (low dosage)+choline (low dosage) | 89.00+4.00^{∗∗} |
| 16 | 2'-FL (medium dosage)+milk fat globule membrane (medium dosage)+choline (medium dosage) | 102.33±3.06^{∗∗} |
| 17 | 2'-FL (high dosage)+milk fat globule membrane (high dosage)+choline (high dosage) | 124.00±5.29^{∗∗} |
| 18 | 2'-FL (low to medium dosage)+milk fat globule membrane (low to medium dosage)+choline (low to medium dosage) | 95.33±3.06^{∗∗} |
| 19 | 2'-FL (medium to high dosage)+milk fat globule membrane (medium to high dosage)+choline (medium to high dosage) | 62.33±5.51^{∗∗} |
| 20 | 2'-FL+milk fat globule membrane+choline | 109.03±8.16^{∗∗} |
| 21 | 2'-FL+milk fat globule membrane+choline | 116.83±9.63^{∗∗} |
| 22 | 2'-FL+milk fat globule membrane+choline | 121.58±7.45^{∗∗} |
| N.B.: | ^{∗}p<0.05, in comparison with the blank control group, | |
| | ^{∗∗}p<0.01, in comparison with the blank control group | |

The results of sample processing showed that all of 2'-FL, milk fat global membrane and choline could increase the number of MAG positive cells, compared with the blank control group (cell experiment example 1), which indicates that these three components help the myelination of oligodendrocyte precursor cell.

However, the inventors found that when any two of the three components were used in combination, there was a strong antagonism.

In particular, it could be seen according to Cell Experiment Examples 12-14 that:
- In terms of the comstituents used and the amount of the constituents used, Cell Experiment Example 12 is equivalent to a combination of Cell Experiment Examples 4 and 6. Relative to the blank control group (Cell Experiment Example 1), Cell Experiment Examples 4 and 6 increased the number of MAG positive cells by 35.66 and 29.66 respectively, while Cell Experiment Example 12 increased the number of MAG positive cells by 25.83, which is not only far less than the sum of the former two (65.32) (a difference from the sum of the former two of Δ1=39.49), but also less than either one of the former two.
- Cell Experiment Example 13 is equivalent to the combination of Cell Experiment Examples 4 and 10. Relative to the blank control group (Cell Experiment Example 1), Cell Experiment Examples 4 and 10 increased the number of MAG positive cells by 35.66 and 53.33 respectively, while Cell Experiment Example 13 increased the number of MAG positive cells by 24.5, which is not only far less than the sum of the former two (88.99) (a difference from the sum of the former two of Δ2=64.49), but also less than either one of the former two.
- Cell Experiment Example 14 is equivalent to the combination of Cell Experiment Examples 6 and 10. However, relative to the blank control group (Cell Experiment Example 1), Cell Experiment Examples 6 and 10 increased the number of MAG positive cells by 29.66 and 53.33 respectively, while Cell Experiment Example 14 increased the number of MAG positive cells by 21.83, which is not only far less than the sum of the former two (82.99) (a difference from the sum of the former two of Δ3=61.16), but also less than either one of the former two.

This indicates that when any two of 2'-FL, milk fat global membrane and choline are used in combination, there is a strong antagonistm in terms of the number of MAG positive cells and thus the myelination of oligodendrocyte precursor cells, and the effect of the use in combination is smaller than that of any one of the components when used alone.

Furthermore, the inventors found that when 2'- FL, milk fat global membrane and choline are used in combination, a significant reduction in inter-component antagonism, and a synergistic increase in the number of MAG positive cells and thus oligodendrocyte precursor cell myelination are enabled.

In particular, in terms of the comstituents used and the amount of the constituents used, Cell Experiment Example 16 is equivalent to the combination of Cell Experiment Examples 7 and 13, or the combination of Cell Experiment Examples 4, 7 and 10. Cell Experiment Examples 4, 7, 10, 13 and 16 increased the number of MAG positive cells by 35.66, 18, 53.33, 24.5 and 61.66 respectively, relative to Cell Experiment Example 1, the increment in the number of MAG positive cells in Cell Experiment Example 16 (61.66) is greater than than the sum of respective increments of Cell Experiment Examples 7 and 13 (42.5), less than the sum of respective increments of Cell Experiment Examples 4, 7 and 10 (106.99) (a difference of Δ4=45.33, less than Δ2=64.49).

In addition, in terms of the comstituents used and the amount of the constituents used, Cell Experiment Example 17 is equivalent to the combination of Cell Experiment Examples 5, 8 and 11. Cell Experiment Examples 5, 8 and 11 increased the number of MAG positive cells by 21.33, 1.66 and 40.66 respectively, relative to Cell Experiment Example 1, while Cell Experiment Example 17 increased the number of MAG positive cells by 83.33, which is greater than the sum of the former three (63.65).

In addition, in terms of the comstituents used and the amount of the constituents used, Cell Experiment Example 20 is equivalent to the combination of Cell Experiment Examples 9 and 12, or the combination of Cell Experiment Examples 4, 6 and 9. Cell Experiment Examples 4, 6, 9, 12 and 20 increased the number of MAG positive cells by 35.66, 29.66, 25.66, 25.83 and 68.36 respectively, relative to Cell Experiment Example 1, the increment realized in Cell Experiment Example 20 (68.36) is greater thant the sum of respective increments of Cell Experiment Examples 9 and 12 (51.49), less than the sum of respective increments of Cell Experiment Examples 4, 6 and 9 (90.98) (a difference of Δ5=22.62, less than Δ1=39.49).

In addition, in terms of the comstituents used and the amount of the constituents used, Cell Experiment Example 21 is equivalent to the combination of Cell Experiment Examples 7 and 13, or the combination of Cell Experiment Examples 4, 7 and 10. Cell Experiment Examples 4, 7, 10, 13 and 21 increased the number of MAG positive cells by 35.66, 18, 53.33, 24.5 and 76.16 respectively, relative to Cell Experiment Example 1, the increment realized in Cell Experiment Example 21 (76.16) is greater than the sum of respective increments of Cell Experiment Examples 7 and 13 (42.5), less than the sum of respective increments of Cell Experiment Examples 4, 7 and 10 (106.99) (a difference of Δ6=30.83, less than Δ2=64.49).

In addition, in terms of the comstituents used and the amount of the constituents used, Cell Experiment Example 22 is equivalent to the combination of Cell Experiment Examples 4 and 14, or the combination of Cell Experiment Examples 6 and 13, or the combination of Cell Experiment Examples 10 and 12, or the combination of Cell Experiment Examples 4, 6 and 10. Relative to the blank control group (Cell Experiment Example 1):
- Cell Experiment Example 22 increased the number of MAG positive cells by 80.91,
- Cell Experiment Examples 4 and 14 increased the number of MAG positive cells by 35.66 and 21.83 respectively, with a sum of 57.49, less than the increment realized in Cell Experiment Example 22 (80.91),
- Cell Experiment Examples 6 and 13 increased the number of MAG positive cells by 29.66 and 24.5 respectively, with a sum of 54.16, less than the increment realized in Cell Experiment Example 22 (80.91),
- Cell Experiment Examples 10 and 12 incresased the number of MAG positive cells by 53.33 and 25.83 respectively, with a sum of 79.16, less than the increment realized in Cell Experiment Example 22 (80.91),
- Cell Experiment Examples 4, 6 and 10 increased the number of MAG positive cells by 35.66, 29.66 and 53.33 respectively, with a sum of 118.65, greater than the increment realized in Cell Experiment Example 22 (80.91), with a difference of Δ7=37.74, less than Δ1=39.49, Δ2=64.49 and Δ3=61.16.

Thus, it can be seen that when 2'-FL, milk fat global membrane and choline are used in combination, the inter-component antagonism may be significantly reduced. There is a synergistic effect among the three components, which can synergistically increase the number of MAG positive cells and therefore myelination of oligodendrocyte precursor cells.

### 2.3 Effects of various samples on OPC cell maturation

In order to measure the effect of mixed nutrition or single nutrition treatment on OPC cell maturation, we evaluated the number of MBP positive cells after 30DIVs. The measured average number of MBP positive cells per well is shown in Table 5.

**Table 5. Effect of different nutrient composition samples containing 2'-FL, milk fat global membrane and/or choline on the number of MBP positive cells**

| Cell Example | Dosage Group | number of MBP positive cells (mean±standard deviation) |
|---|---|---|
| 1 | blank control group | 62.33±4.51 |
| 2 | positive control group Olesoxime | 124.00±3.61^{∗∗} |
| 3 | 2'-FL (low dosage) | 104.00±4.58^{∗∗} |
| 4 | 2'-FL (medium dosage) | 104.67±5.03^{∗∗} |
| 5 | 2'-FL (high dosage) | 83.00±5.29^{∗∗} |
| 6 | milk fat globule membrane (low dosage) | 106.67±5.51^{∗∗} |
| 7 | milk fat globule membrane (medium dosage) | 92.00±6.24^{∗∗} |
| 8 | milk fat globule membrane (high dosage) | 52.33±6.11^{∗} |
| 9 | choline (low dosage) | 113.33±5.69^{∗∗} |
| 10 | choline (medium dosage) | 133.67±4.16^{∗∗} |
| 11 | choline (high dosage) | 68.00±2.65 |
| 12 | 2'-FL+milk fat globule membrane | 82.50±6.74^{∗∗} |
| 13 | 2'-FL+choline | 80.83±8.15^{∗∗} |
| 14 | milk fat globule membrane+choline | 78.33±5.95^{∗} |
| 15 | 2'-FL (low dosage)+milk fat globule membrane (low dosage)+choline (low dosage) | 123.00±6.24^{∗∗} |
| 16 | 2'-FL (medium dosage)+milk fat globule membrane (medium dosage)+choline (medium dosage) | 140.67±6.03^{∗∗} |
| 17 | 2'-FL (high dosage)+milk fat globule membrane (high dosage)+choline (high dosage) | 156.33±5.51^{∗∗} |
| 18 | 2'-FL (low to medium dosage)+milk fat globule membrane (low to medium dosage)+choline (low to medium dosage) | 127.67 ±5.86^{∗∗} |
| 19 | 2'-FL (medium to high dosage)+milk fat globule membrane (medium to high dosage)+choline (medium to high dosage) | 93.00±9.54^{∗∗} |
| 20 | 2'-FL+milk fat globule membrane+choline | 136.4±6.38^{∗∗} |
| 21 | 2'-FL+milk fat globule membrane+choline | 155.58±6.92^{∗∗} |
| 22 | 2'-FL+milk fat globule membrane+choline | 161.78±7.85^{∗∗} |
| N.B.: | ^{∗}p<0.05, in comparison with the blank control group, | |
| | ^{∗∗}p<0.01, in comparison with the blank control group | |

The sample treatment results showed that, relative to the blank control group (cell experiment example 1), each one of 2'-FL, milk fat global membrane, and choline can increase the number of MBP positive cells, which indicates that these three ingredients can help the maturation of the oligodendrocyte precursor cells.

However, the inventors found that when any two of the three components were used together, there was a strong antagonism.

In particular, it can be seen according to Cell Experiment Examples 12-14 that:
- In terms of the comstituents used and the amount of the constituents used, Cell Experiment Example 12 is equivalent to a combination of Cell Experiment Examples 4 and 6. Relative to the blank control group (Cell Experiment Example 1), Cell Experiment Examples 4 and 6 increased the number of MBP positive cells by 42.34 and 44.34 respectively, while Cell Experiment Example 12 increased the number of MBP positive cells by 20.17, which is not only far less than the sum of the former two (86.68) (a difference from the sum of the former two of Δ1=66.51), but also less than either one of the former two.
- Cell Experiment Example 13 is equivalent to the combination of Cell Experiment Examples 4 and 10. Relative to the blank control group (Cell Experiment Example 1), Cell Experiment Examples 4 and 10 increased the number of MBP positive cells by 42.34 and 71.34 respectively, while Cell Experiment Example 13 increased the number of MBP positive cells by 18.5, which is not only far less than the sum of the former two (113.68) (a difference from the sum of the former two of Δ2=95.18), but also less than either one of the former two.
- Cell Experiment Example 14 is equivalent to the combination of Cell Experiment Examples 6 and 10. However, relative to the blank control group (Cell Experiment Example 1), Cell Experiment Examples 6 and 10 increased the number of MBP positive cells by 44.34 and 71.34 respectively, , while Cell Experiment Example 14 increased the number of MBP positive cells by 16, which is not only far less than the sum of the former two (115.68) (a difference from the sum of the former two of Δ3=99.68), but also less than either one of the former two.

This indicates that when any two of 2'-FL, milk fat global membrane and choline are used in combination, there is a strong antagonism in terms of the number of MBP positive cells and thus the maturation of oligodendrocyte precursor cells, and the effect of their use in combination is less than that when any one of the components is used alone.

The inventors further found that when 2'-FL, milk fat global membrane and choline are used in combination, a significant reduction in inter-component antagonism, and a synergistic increase in the number of MBP positive cells and thus maturation of oligodendrocyte precursor cells.

In particular, in terms of the comstituents used and the amount of the constituents used, Cell Experiment Example 16 is equivalent to the combination ot Cell Experiment Examples 7 and 13, or the combination of Cell Experiment Examples 4, 7 and 10. Cell Experiment Examples 4, 7, 10, 13 and 16 increased the number of MBP positive cells by 42.34, 29.67, 71.34, 18.5 and 78.34 respectively, relative to Cell Experiment Example 1, the increment in MBP positive cells in Cell Experiment Example 16 (78.34) is greater than the sum of respective increments of Cell Experiment Examples 7 and 13 (48.17), less than the sum of respective increments of Cell Experiment Examples 4, 7 and 10 (143.35) (a difference of Δ4=65.01, less than Δ2=95.18).

In addition, in terms of the comstituents used and the amount of the constituents used, Cell Experiment Example 17 is equivalent to the combination of Cell Experiment Examples 5, 8 and 11. Cell Experiment Examples 5, 8 and 11 increased the number of MBP positive cells by 20.67, -10 and 5.67 respectively, relative to Cell Experiment Example 1, while Cell Experiment Example 17 increased the number of MBP positive cells by 94, which is greater than the sum of the former three (16.34).

In addition, in terms of the comstituents used and the amount of the constituents used, Cell Experiment Example 20 is equivalent to the combination of Cell Experiment Examples 9 and 12, or the combination of Cell Experiment Examples 4, 6 and 9. Cell Experiment Examples 4, 6, 9, 12 and 20 increased the number of MBP positive cells by 42.34, 44.34, 51, 20.17 and 74.07 respectively, relative to Cell Experiment Example 1, the Increment realized in Cell Experiment Example 20 (74.07) is greater than the sum of respective increments of Cell Experiment Examples 9 and 12 (71.17), less than the sum of respective increments of Cell Experiment Examples 4, 6 and 9 (137.68) (a difference of Δ5=63.61, less than Δ1=66.51).

In addition, in terms of the comstituents used and the amount of the constituents used, Cell Experiment Example 21 is equivalent to the combination of Cell Experiment Examples 7 and 13, or the combination of Cell Experiment Examples 4, 7 and 10. Cell Experiment Examples 4, 7, 10, 13 and 21 increased the number of MBP positive cells by 42.34, 29.67, 71.34, 18.5 and 93.25 respectively, relative to Cell Experiment Example 1, the increment realized in Cell Experiment Example 21 (93.25) is greater than the sum of respective increments of Cell Experiment Examples 7 and 13 (48.17), less than the sum of respective increments of Cell Experiment Examples 4, 7 and 10 (143.35) (a difference of Δ6=50.1, less than Δ2=95.18).

In addition, in terms of the comstituents used and the amount of the constituents used, Cell Experiment Example 22 is equivalent to the combination of Cell Experiment Examples 4 and 14, or the combination of Cell Experiment Examples 6 and 13, or the combination of Cell Experiment Examples 10 and 12, or the combination of Cell Experiment Examples 4, 6 and 10. Relative to the blank control group (Cell Experiment Example 1):
- Cell Experiment Example 22 increased the number of MBP positive cells by 99.45,
- Cell Experiment Examples 4 and 14 increased the number of MBP positive cells by 42.34 and 16 respectively, with a sum of 58.34, less than the increment realized in Cell Experiment Example 22 (99.45),
- Cell Experiment Examples 6 and 13 increased the number of MBP positive cells by 44.34 and 18.5 respectively, with a sum of 62.84, less than the increment realized in Cell Experiment Example 22 (99.45),
- Cell Experiment Examples 10 and 12 incresed the number of MBP positive cells by 71.34 and 20.17 respectively, with a sum of 91.51, less than the increment realized in Cell Experiment Example 22 (99.45),
- Cell Experiment Examples 4, 6 and 10 increased the number of MBP positive cells by 42.34, 44.34 and 71.34 respectively, with a sum of 158.02, greater than the increment realized in Cell Experiment Example 22 (99.45), with a difference of Δ7=58.57, less than Δ1=66.51, Δ2=95.18 and Δ3=99.68.

Thus, it can be seen that when the three of 2'-FL, milk fat global membrane and choline are used in combination, a significant reduction in inter-component antagonism is enabled. There is a synergistic effect among the three components, which can synergistically increase the number of MBP positive cells and thus the maturation of oligodendrocyte precursor cells.

The present invention provides a research concerning a composition of a human milk oligosaccharide (preferably neural fucosylated human milk oligosaccharide (HMO), which is preferably one or more selected from the group consisting of 2'-fluorosyl lactose (2'-FL), 3'-fluorosyl lactose (3'-FL), lacto-N-fucopentaose I (LNFP I), lacto-N-difucoexaose I (LNDFH I), lacto-N-difucoexaose II (LNDFH II), preferably 2'-fluorosyl lactose (2'-FL)), milk fat globule membrane, and choline or edible choline on brain development, especially on neurodevelopment, in particular on oligodendrocyte precursor cell (OPC) proliferation, differentiation or maturation into ligodendrocyte (OL) and the myelination characteristics of oligodendrocyte (OL). It provides a new idea for the development of functional food in the future. Human milk oligosaccharide (preferably neurtral fucosylated human milk oligosaccharide (HMO), which is preferably one or more selected from the group consisting of 2'-fucosyl lactose (2'-FL), 3'-fucosyl lactose (3'-FL), lacto-N-fucopentaose I (LNFP I), lacto-N-difucohexaose I (LNDFH I), lacto-N-difucohexaose II (LNDFH II), preferably 2'-fucosyl lactose (2'-FL)), milk fat globule membrane and choline or edible choline has a broad prospect in terms of improving memory and brain development. It is found that during the development of central nervous system, extracellular environment plays an important role in regulating brain homeostasis and controlling myelin formation and other cellular mechanisms. Deficiency of key nutrients will significantly affect brain development. Our research shows that in *in vitro* models, brain cell cultures treated with a combination of 2'-FL, milk fat globle membrane and choline or edible choline increase OPC numbers, differentiation or maturion into OL, and the myelination characteristics of OL. There is a good inter-component synergistic effect.

### Application Example

All "Part(s)" in the following application examples are parts by weight. A % content of an ingredient is a % by weight content.

In addition, in each of the following application examples, the following raw material sources are used unless otherwise stated.
Raw milk: Heilongjiang Feihe Dairy Co., Ltd
Whole milk powder: Heilongjiang Feihe Dairy Co., Ltd
Skimmed milk powder: Kerry, Ireland
2'-fucosyl lactose (2'-fucosyl lactose 96.0%): GlyCare ^{™} 2'-FL 9000
Concentrated whey protein powder rich in milk fat globule membrane (milk fat globle membrane 7%): Lacprodan^{®} MFGM-10, Denmark
Milk fat globle membrane powder (phospholipid 12.3%): Tatua of New Zealand PLC1
Choline chloride (choline content 72.5%): Beijing Jinkangpu Food Technology Co., Ltd
Desalinated whey powder: Euroserum (Red Bird), France
Oligogalactose: Baolingbao Biology Co., Ltd
Mixed vegetable oil: Cargill Cereals and Oils (Nantong) Co., Ltd
Mixed vegetable oil (containing 1,3-dioleate-2-palmitate triglyceride): Bunge Loders Croklaan (Xiamen) Oil Technology Co., Ltd
Formulated vitamin: DSM Vitamin (Shanghai) Co., Ltd
Formulated minerals: DSM Vitamin (Shanghai) Co., Ltd
Solid corn syrup: Baolingbao Biology Co., Ltd
Oligofructose: Baolingbao Biology Co., Ltd
Oligoisomaltose: Baolingbao Biology Co., Ltd

### Application Example 1

A formula milk powder for infants, containing 2'-fucosyl lactose, milk fat globule membrane and choline, is prepared, per 1000 parts of the formula milk powder, by the following constituents in parts by weight:
the present milk powder uses the raw materials of: 195 parts of raw milk (on a dry basis) (each part contains 0.10% choline, and contains 0.20 % milk fat global membrane), 28.5 parts of concentrated whey protein powder rich in milk fat global membrane (each part contains 7% milk fat global membrane), 10.42 parts of 2'-fluorosyl lactose (96.0% milk fat global membrane in each part), 0.3 parts of choline chloride (72.5% choline in each part), 400 parts of desalinated whey powder (0.15% choline contained in each part), 83.78 parts of lactose, 37 parts of oligogalactose, 200 parts of mixed vegetable oil, 16 parts of arachidonic acid oil powder (10%), 14 parts of docosahexaenoic acid oil powder (7%), 2 parts of sodium citrate, 2 parts of potassium chloride, 6 parts of calcium citrate, 3 parts of formulated vitamin, and 2 parts of formulated minerals; after the above raw materials have been mixed evenly, they underwent pasteurization, homogenization, evaporation and concentration, and spray drying, forming powder-like semi-finished products. The evenly mixed milk powder is packed with filled-in nitrogen to obtain the final product.

### Application Example 2

A formula milk powder for infants, containing 2'-fucosyl lactose, milk fat globule membrane and choline, is prepared, per 1000 parts of the formula milk powder, by the following constituents in parts by weight:
the present milk powder uses the raw materials of: 220 parts of raw milk (on a dry basis) (each part contains 0.10% choline, and contains 0.20% of milk fat global membrane), 15 parts of concentrated whey protein powder rich in milk fat global membrane (7% milk fat global membrane in each part), 10.42 parts of 2'-fluorosyl lactose (96.0% of 2'-fluorosyl lactose in each part), 3.1 parts of choline chloride (72.5% choline in each part), 400 parts of desalinated whey powder (0.15% choline contained in each part), 74.48 parts of lactose, 37 parts of oligolactose, 195 parts of mixed vegetable oil, 16 parts of arachidonic acid oil powder (10%), 14 parts of docosahexaenoic acid oil powder (7%), 2 parts of sodium citrate, 2 parts of potassium chloride, 6 parts of calcium citrate, 3 parts of formulated vitamin, 2 parts of formulated minerals; after the above raw materials have been mixed evenly, they underwent pasteurization, homogenization, evaporation and concentration, and spray drying, forming powder-like semi-finished products. The evenly mixed milk powder is packed with filled-in nitrogen to obtain the final product.

### Application Example 3

A formula milk powder for young children, containing 2'-fucosyl lactose, milk fat globule membrane and choline, is prepared, per 1000 parts of the formula milk powder, by the following constituents in parts by weight:
the present milk powder uses the raw materials of: 220 parts of raw milk (on a dry basis) (each part contains 0.10% choline, and contains 0.20% milk fat global membrane), 15 parts of concentrated whey almubin powder rich in milk fat global membrane (7% milk fat global membrane in each part), 10.42 parts of 2'-fluorosyl lactose (96.0% of 2'-fluorosyl lactose in each part), 1.5 parts of choline chloride (72.5% choline contained in each part), 380 parts of desalinated whey powder (0.15% choline contained in each part), 120 parts of skimmed milk powder (0.14% choline in each part), 72.43 parts of lactose, 40 parts of oligolactose, 120 parts of mixed vegetable oil (containing 1,3-dioleate 2-palmitate triglyceride), 4.5 parts of arachidonic acid oil powder (10%), 6.5 parts of docosahexaenoic acid oil powder (7%), 5 parts of calcium citrate, 3 parts of formulated vitamin, 1 part of formulated minerals, and 0.65 parts of formulated nucleotides; after the above raw materials have been mixed evenly, they underwent pasteurization, homogenization, evaporation and concentration, and spray drying, forming powder-like semi-finished products. The evenly mixed milk powder is packed with filled-in nitrogen to obtain the final product.

### Application Example 4

A formulated milk powder, containing 2'-fucosyl lactose, milk fat globule membrane and choline, suitable for pregnant women, is prepared, per 1000 parts of the formulated milk powder, by the following constituents in parts by weight:
the present milk powder uses the raw materials of: 436 parts of whole milk powder (each part contains 0.10% choline, and contains 0.20% milk fat globle membrane), 40.65 parts of milk fat globle membrane powder (12.3% milk fat globle membrane in each part), 10.42 parts of 2'-fucosyl lactose (96.0% 2'-fucosyl lactose in each part), 2.5 parts of choline chloride (72.5% choline in each part), 200 parts of skimmed milk powder (0.14% choline contained in each part), 73.93 parts of lactose, 165 parts of maltodextrin, 60 parts of oligoisomaltose, 7 parts of DHA powder (7%), 3.5 parts of formulated vitamins, 1 part of formulated mineral; after the above raw materials have been mixed evenly, they underwent pasteurization, homogenization, evaporation and concentration, and spray drying, forming powder-like semi-finished products. The evenly mixed milk powder is packed with filled-in nitrogen to obtain the final product.

### Application Example 5

A formulated milk powder, containing 2'-fucosyl lactose, milk fat globule membrane and choline, suitable for pregnant women, is prepared, per 1000 parts of the formulated milk powder, by the following constituents in parts by weight: The present milk powder uses the raw materials of: 436 parts of raw milk (on a dry basis) (each part constains 0.10% choline, and contains 0.20% milk fat global membrane), 5 parts of milk fat global membrane powder (12.3% milk fat global membrane in each part), 10.42 parts of 2'-fluorosyl lactose (96.0% 2'-fluorosyl lactose in each portion), 1.8 parts of choline chloride (72.5% choline in each part), 250 parts of skimmed milk powder (0.14% choline contained in each part), 78.28 parts of lactose, 137 parts of maltodextrin, 70 parts of oligoisomaltose, 7 parts of DHA powder (7%), 3.5 parts of formulated vitamin, 1 part of formulated mineral; after the above raw materials have been mixed evenly, they underwent pasteurization, homogenization, evaporation and concentration, and spray drying, forming powder-like semi-finished products. The evenly mixed milk powder is packed with filled-in nitrogen to obtain the final product.

### Application Example 6

A formulated milk powder, containing 2'-fucosyl lactose, milk fat globule membrane and choline, suitable for pregnant women, and is prepared, per 1000 parts of the formulated milk powder, by the following constituents in parts by weight:
the present milk powder uses the raw materials of: 436 parts of whole milk powder (each part contains 0.10% choline, and contains 0.2% milk fat globle membrane), 35 parts of milk fat globle membrane powder (12.3% milk fat globle membrane in each part), 10.43 parts of 2'-fucosyl lactose (96.0% 2'-fucosyl lactose in each part), 3.1 parts of choline chloride (72.5% choline in each part), 210 parts of skimmed milk powder (0.14% choline contained in each part), 65.98 parts of lactose, 168 parts of maltodextrin, 60 parts of oligoisomaltose, 7 parts of DHA powder (7%), 3.5 parts of formulated vitamin, 1 part of formulated mineral; after the above raw materials have been mixed evenly, they underwent pasteurization, homogenization, evaporation and concentration, and spray drying, forming powder-like semi-finished products. The evenly mixed milk powder is packed with filled-in nitrogen to obtain the final product.

What is described above is only an exemplary embodiment of the present invention. It should be pointed out here that, for those of ordinary skills in the art, modifications can be made without departing from the scope of the present invention, yet all these belong to the scope of the present invention.

## Claims

1. A nutrient composition comprising:
- human milk oligosaccharide (HMO), preferably neurtral fucosylated human milk oligosaccharide (HMO), preferably one or more selected from the group consisting of 2'-fucosyl lactose (2'-FL), 3'-fucosyl lactose (3'-FL), lacto-N-fucopentaose I (LNFP I), lacto-N-difucohexaose I (LNDFH I), and lacto-N-difucohexaose II (LNDFH II), preferably 2'-fucosyl lactose;
- milk fat globule membrane; and
- choline and/or edible choline derivative, preferably the edible choline derivative being selected from the group consisting of choline chloride and/or choline bitartrate.

2. The nutrient composition of claim 1, consisting of the human milk oligosaccharide; the milk fat globule membrane; and the choline and/or edible choline derivative.

3. The nutrient composition of either of claims 1 and 2, wherein the human milk oligosaccharide is provided in a form of natural origin, and/or synthetic origin, and/or bacterial fermentation origin.

4. The nutrient composition of any one of claims 1 to 3, wherein:
the milk fat globule membrane is cow and/or sheep milk-origin phospholipid; and/or
the milk fat globule membrane is provided in the form of an protein powder containing milk fat globule membrane, said protein powder containing milk fat globule membrane preferably having a milk fat globule membrane content of 6~25% by weight; and/or of a cow milk-origin phospholipid, said cow milk-origin phospholipid preferably having a milk fat globule membrane content of 9~60% by weight; and/or
the milk fat globule membrane at least contains sphingophospholipid and phosphatidylcholine, and optionally further contains phosphatidylserine and/or phosphatidylethanolamine, wherein sphingophospholipid represents 10% by weight or more of the total milk fat globule membrane, and phosphatidylcholine represents 15% by weight or more of the total milk fat globule membrane.

5. The nutrient composition of any one of claims 1 to 4, wherein the choline and/or edible choline derivative is a choline chloride and/or choline bitartrate.

6. The nutrient composition of any one of claims 1 to 5, wherein:
a mass ratio of the human milk oligosaccharide to the milk fat globule membrane is 1:0.01-500, preferably 1:0.02-100, preferably 1:0.05-50, preferably 1:0.1-10, preferably 1:0.15-6; and
a mass ratio of the human milk oligosaccharide to the choline and/or edible choline derivative is 1:0.001-100, preferably 1:0.002-20, preferably 1:0.005-5, preferably 1:0.01-1, preferably 1:0.02-0.5, as a ratio of a mass of the human milk oligosaccharide to an equivalent choline total mass;
preferably, a mass ratio of the human milk oligosaccharide to the milk fat globule membrane to the choline and/or edible choline derivative is 1:0.01-500:0.001-100, preferably 1:0.02-100:0.002-20, preferably 1:0.05-50:0.005-5, preferably 1:0.1-10:0.01-1, preferably 1:0.15-6:0.02-0.5, as a ratio of a mass of the human milk oligosaccharide to a mass of the milk fat globule membrane to the equivalent choline total mass.

7. A food comprising the nutrient composition of any one of claims 1 to 6, the food preferably being in the form of powder or liquid.

8. The food of claim 7, which is a formula food for infants and young children, for example a formula food for infants, a formula food for more grown-up infants, and a formula food for young children, for example a formula milk powder for infants and young children, for example a formula milk powder for infants, and a formula milk powder for young children; a complementary food for infants; nutritional or dietary supplements; or a formulated milk powder for pregnant women.

9. The food of any one of claims 7 to 8, wherein the amount of said nutrient composition added, relative to the total weight of said food, is such that:
the human milk oligosaccharide is at a content by weight of at least 0.01%, preferably at least 0.05%, preferably at least 0.1% and at most 10.0%, preferably at most 5.0%, preferably at most 1.0%,
the milk fat globule membrane is at a content by weight of at least 0.01%, preferably at least 0.05%, preferably at least 0.1% and at most 5.0%, preferably at most 1.0%, preferably at most 0.6%,
choline or edible choline is at a content by weight of at least 0.01%, preferably at least 0.05%, preferably at least 0.1% and at most 3.0%, preferably at most 1.0%, preferably at most 0.3%, wherein a mass of the edible choline is converted into a mass of choline.

10. Use of the nutrient composition of any one of claims 1 to 6 or the food of any one of claims 7 to 9 for non-curing purposes of improving the brain development and intelligence of infants and young children, especially promoting neurodevelopment such as neuron maturation, synaptogenesis and myelination, in particular promoting the the oligodendrocyte precursor cell proliferation, maturation, and differentiation into oligodendrocytes and/or myelination of oligodendrocytes.
